# EUROPEAN PATENT APPLICATION

(11) **EP 4 477 223 A1**
(43) Date of publication of application: **18.12.2024**
(21) Application number: 23749933.0
(22) Date of filing: 30.01.2023
(51) Int. Cl.: A61K 31/519, A61K 31/426, A61P 13/12

(54) **PHARMACEUTICAL COMPOSITION COMPRISING ALLOPURINOL, FEBUXOSTAT OR PHARMACEUTICALLY ACCEPTABLE SALT THEREOF FOR PREVENTION OR TREATMENT OF CHRONIC KIDNEY DISEASE IN SUBJECT HAVING HIGH BLOOD URIC ACID CONCENTRATION**

(30) Priority: 07.02.2022 KR 20220015279
(71) Applicant: Indream Healthcare Inc., Jeju-si, Jeju-do 63208 (KR)
(72) Inventor: GHANG, Byeong Zu, Seogwipo-si, Jeju-do 63610 (KR)
(74) Representative: Murgitroyd & Company
(86) International application number: PCT/KR2023/001331
(87) International publication number: WO 2023/149700

(57) **Abstract**

The present invention provides a pharmaceutical composition comprising as an active ingredient a therapeutically effective amount of allopurinol, febuxostat or a pharmaceutically acceptable salt thereof for the prevention or treatment of chronic kidney disease in a subject having a high serum uric acid concentration, wherein when administered to a subject having a serum uric acid level of 6 mg/dl or more, the pharmaceutical composition reduces the subject's serum uric acid level to less than 6 mg/dl. Therefore, the pharmaceutical composition according to the present invention exhibits an excellent effect on xanthine oxidase inhibitors, and thus can effectively treat or prevent chronic kidney disease by being administered to a selected subject.

## Description

### BACKGROUND OF THE INVENTION

### Technical Field

The present disclosure relates to a pharmaceutical composition including allopurinol, febuxostat, or a pharmaceutically acceptable salt thereof, and more particularly, to a pharmaceutical composition for use in preventing or treating chronic kidney disease of a subject having a high serum uric acid concentration.

### Background Art

Chronic kidney disease is one of the leading causes of death in most countries. There have been attempts to prevent or treat the chronic kidney disease using a xanthine oxidase inhibitor, but treatment efficacy for the chronic kidney disease of the xanthine oxidase inhibitor has not been clearly revealed. Multiple studies have reported that the xanthine oxidase inhibitor rather worsens a kidney function. For example, "Acute kidney injury associated with febuxostat and allopurinol: a post-marketing study, Arthritis Research & Therapy volume 21, Article number: 229 (2019)" mentions that acute renal failure was reported 5.7 times and 3.3 times more often with febuxostat and allopurinol, "Drug-Induced Nephrotoxicity, Am Fam Physician. 2008 Sep" mentions that allopurinol causes acute interstitial nephritis, and also, "Allopurinol-induced severe hypersensitivity with acute renal failure, Kaohsiung J Med Sci. 2005 May;21(5):228-32" mentions that allopurinol worsens kidney function. In addition, since a method of treating chronic kidney disease by administering the xanthine oxidase inhibitor is not clinically recommended at all and there is no approval for kidney disease, the treatment method has not been attempted at all.

### SUMMARY OF THE INVENTION

### Technical Goals

The present disclosure is directed to providing prevention or treatment of chronic kidney disease by administering a pharmaceutical composition including a therapeutically effective amount of allopurinol, febuxostat, or a pharmaceutically acceptable salt thereof as an active ingredient to a subject having a high serum uric acid concentration.

In particular, the present disclosure is directed to providing treatment or prevention of chronic kidney disease by administering the pharmaceutical composition according to the present disclosure to a subject selected for responding with an excellent effect when treated with allopurinol, febuxostat, or a pharmaceutically acceptable salt thereof.

### Technical solutions

According to one exemplary embodiment of the present disclosure, a pharmaceutical composition for use in preventing or treating chronic kidney disease includes a therapeutically effective amount of allopurinol, febuxostat, or a pharmaceutically acceptable salt thereof as an active ingredient. The composition is administered to a subject having a serum uric acid concentration of 6 milligrams per deciliter (mg/dl) or more to decrease the serum uric acid concentration of the subject to less than 6 mg/dl.

The subject may have a blood glucose level of 126 mg/dl or more, a diastolic blood pressure of 80 millimeters of mercury (mmHg) or lower, or a systolic blood pressure of 130 mmHg or lower.

The subject may have the blood glucose level of 126 mg/dl or more.

The subject may have the diastolic blood pressure of 80 mmHg or lower and the systolic blood pressure of 130 mmHg or lower.

The active ingredient may be the allopurinol or the pharmaceutically acceptable salt thereof, and the subject may not have congestive cardiac failure.

The active ingredient may be the febuxostat or the pharmaceutically acceptable salt thereof, and the subject may have the serum uric acid concentration of 8 mg/dl or more.

The subject may have the serum uric acid concentration of 7 mg/dl or more.

The subject may have hyperuricacidemia or gout.

The subject may have gout.

The active ingredient may be the febuxostat or the pharmaceutically acceptable salt thereof, and the gout may have lasted 10 years or more.

The active ingredient may be the allopurinol or the pharmaceutically acceptable salt thereof, and a dosage of the active ingredient may be 100 milligrams (mg) to 600 mg per day based on the amount of the allopurinol. For example, the dosage of the allopurinol or the pharmaceutically acceptable salt may be 100 mg, 200 mg, 300 mg, 400 mg, 500 mg, or 600 mg per day based on the amount of the allopurinol.

The active ingredient may be febuxostat or the pharmaceutically acceptable salt thereof, and the dosage of the active ingredient may be 20 mg to 120 mg per day based on the amount of the febuxostat. For example, the dosage of the febuxostat or the pharmaceutically acceptable salt may be 20 mg, 40 mg, 60 mg, 80 mg, 100 mg, or 120 mg per day based on the amount of the febuxostat.

The subject may have an estimated glomerular filtration rate (eGFR) of 45 milliliters per minute per 1.73 square meters (ml/min/1.73 m²) or more.

The active ingredient may be the allopurinol or the pharmaceutically acceptable salt thereof. The active ingredient may be administered to the subject having the eGFR of 60 ml/min/1.73 m² or more at a dose of 300 mg per day based on the amount of the allopurinol, and if the serum uric acid concentration of the subject is not decreased to less than 6 mg/dl at one month after the administration, the active ingredient may be administered at a dose of 600 mg per day based on the amount of the allopurinol, and the active ingredient may be administered to the subject having the eGFR of 30 ml/min/1.73 m² or more and less than 60 ml/min/1.73 m² at a dose of 200 mg per day based on the amount of the allopurinol, and if the serum uric acid concentration of the subject is not decreased to less than 6 mg/dl at one month after the administration, the active ingredient may be administered at a dose which increased in increments of 100 mg per month up to a maximum of 400 mg per day based on the amount of the allopurinol.

The active ingredient may be the febuxostat or the pharmaceutically acceptable salt thereof. The active ingredient may be administered at a dose of 40 mg per day based on the amount of the febuxostat, and if the serum uric acid concentration of the subject is not decreased to less than 6 mg/dl at 2 weeks after the administration, the active ingredient may be administered at a dose of 80 mg per day based on the amount of the febuxostat.

The pharmaceutical composition may further include a pharmaceutically acceptable additive or carrier.

According to another exemplary embodiment of the present disclosure, a method for preventing or treating chronic kidney disease includes administering a pharmaceutical composition including a therapeutically effective amount of allopurinol, febuxostat, or a pharmaceutically acceptable salt thereof as an active ingredient to a subject having a serum uric acid concentration of 6 mg/dl or more to decrease the serum uric acid concentration of the subject to less than 6 mg/dl.

According to another exemplary embodiment of the present disclosure, a use of a composition including a therapeutically effective amount of allopurinol, febuxostat, or a pharmaceutically acceptable salt thereof, in preparation of a drug for preventing or treating chronic kidney disease by decreasing a serum uric acid concentration of a subject having the serum uric acid concentration of 6 mg/dl or more to less than 6 mg/dl is provided.

According to still another exemplary embodiment of the present disclosure, a use of a composition including a therapeutically effective amount of allopurinol, febuxostat, or a pharmaceutically acceptable salt thereof as an active ingredient for decreasing a serum uric acid concentration of a subject having the serum uric acid concentration of 6 mg/dl or more to less than 6 mg/dl, for preventing or treating chronic kidney disease is provided.

### Effects of the Invention

According to one exemplary embodiment, chronic kidney disease may be prevented or treated by administering a pharmaceutical composition including a therapeutically effective amount of allopurinol, febuxostat, or a pharmaceutically acceptable salt thereof as an active ingredient to a subject having a high serum uric acid concentration.

In addition, according to one exemplary embodiment, chronic kidney disease may be prevented or treated more effectively by selecting a subject having an excellent effect from a pharmaceutical composition including a therapeutically effective amount of allopurinol, febuxostat, or a pharmaceutically acceptable salt thereof as an active ingredient.

### BRIEF DESCRIPTION OF THE DRAWINGS

FIG. 1A is a graph showing the change in eGFR over time for each patient group classified depending on the serum uric acid concentration in an allopurinol administration group having a blood glucose level of 126 mg/dl or more over time.
FIG. 1B is a graph showing the change in eGFR over time for each patient group classified depending on the serum uric acid concentration in an allopurinol administration group having a systolic blood pressure of 130 mmHg or lower.
FIG. 1C is a graph showing the change in eGFR over time for each patient group classified depending on the serum uric acid concentration in an allopurinol administration group having a diastolic blood pressure of 80 mm/hg or lower.
FIG. 1D is a graph showing the change in eGFR over time for each patient group classified depending on the serum uric acid concentration in an allopurinol administration group having no congestive cardiac failure history.
FIG. 2A is a graph showing the change in eGFR over time for each patient group classified depending on the serum uric acid concentration in a febuxostat administration group having a blood glucose level of 126 mg/dl or more.
FIG. 2B is a graph showing the change in eGFR over time for each patient group classified depending on the serum uric acid concentration in a febuxostat administration group having a systolic blood pressure of 130 mm/hg or lower.
FIG. 2C is a graph showing the change in eGFR over time for each patient group classified depending on the serum uric acid concentration in a febuxostat administration group having a diastolic blood pressure of 80 mm/hg or lower.
FIG. 2D is a graph showing the change in eGFR over time for each patient group classified depending on the serum uric acid concentration in a febuxostat administration group having a serum uric acid concentration of 8 mg/dl or more.
FIG. 2E is a graph showing the change in eGFR over time for each patient group classified depending on the serum uric acid concentration in a febuxostat administration group having a gout history of 10 years or more.
FIG. 3A is a graph showing the change in eGFR over time for each patient group classified depending on the serum uric acid concentration in the allopurinol or febuxostat administration group having the blood glucose level of 126 mg/dl or more.
FIG. 3B is a graph showing the change in eGFR over time for each patient group classified depending on the serum uric acid concentration in the allopurinol or febuxostat administration group having the systolic blood pressure of 130 mm/hg or lower.
FIG. 3C is a graph showing the change in eGFR over time for each patient group classified depending on the serum uric acid concentration in the allopurinol or febuxostat administration group having the diastolic blood pressure of 80 mm/hg or lower.

### DETAILED DESCRIPTION OF THE EMBODIMENTS

Unless otherwise defined, all technical and scientific terms used in the present specification have the same meaning as commonly understood by a person skilled in the art to which the present disclosure pertains. In general, the terminology used in the present specification is well-known and commonly used in the art.

The term "and/or" in the present specification is used as a term referring to combinations of all configurations derived from a plurality of constituent elements.

According to one exemplary embodiment of the present disclosure, a pharmaceutical composition for use in preventing or treating chronic kidney disease including a therapeutically effective amount of allopurinol, febuxostat, or a pharmaceutically acceptable salt thereof as an active ingredient is provided. In the present specification, a pharmaceutically acceptable salt thereof may refer to a pharmaceutically acceptable salt of allopurinol or a pharmaceutically acceptable salt of febuxostat.

Allopurinol and febuxostat are drugs used for hyperuricacidemia patients, and since they may inhibit a xanthine oxidase, they inhibit conversion of hypoxanthine or xanthine into uric acid, and thus, decrease a serum uric acid concentration and decrease deposition of uric acid in, for example, bone and joints.

Structures of the allopurinol and the febuxostat are as follows:

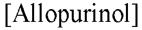

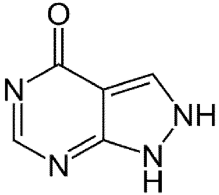

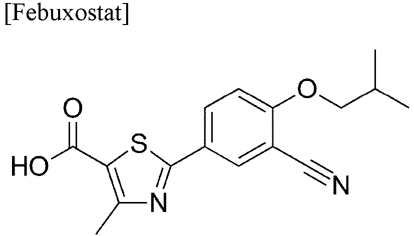

Allopurinol and febuxostat are used for preventing or treating hyperuricacidemia or gout, but the therapeutic effectiveness for chronic kidney disease has not been clearly revealed at this time, and rather, multiple studies are being reported that they make kidney diseases worse, and thus, a method for treating chronic kidney disease using allopurinol and febuxostat is not clinically attempted at all.

Therefore, it is important to confirm a serum uric acid concentration range where patients with kidney diseases may have an effective prevention or therapeutic effect by administering a xanthine oxidase inhibitor such as allopurinol or febuxostat. In addition, it is important to select a patient group who respond with a particularly excellent effect when treated with a xanthine oxidase inhibitor such as allopurinol and febuxostat to selectively treat the subjects who has an effect from allopurinol and febuxostat.

Chronic kidney disease (CKD) is a disease in which the ability of a kidney to filter metabolic wastes in blood gradually declines. The chronic kidney disease includes chronic kidney failure or chronic kidney disorder.

The chronic kidney disease may be stratified into five disease stages depending on a glomerular filtration rate (GFR, ml/min/1.73 m²). A 1st stage is characterized by a normal GFR of 90 or more and continuous albuminuria (for example, urine albumin creatinine ratio (UACR) ≥ 30 mg/g) or known structural or genetic kidney disease. A 2nd stage is characterized by mild kidney disorder showing a low GFR decrease (GFR of 60 to 89), a 3rd stage is characterized by moderate kidney disorder showing a middle GFR decrease (GFR of 30 to 59), a 4th stage is characterized by severe kidney disorder showing a high GFR decrease (GFR of 15 to 29), and a 5th stage, which is the final stage, is characterized by established renal failure (end-stage renal disease, ESRD) requiring dialysis or showing a GFR of less than 15.

Therefore, the chronic kidney disease and its stages (CKD 1 to 5) may be confirmed or classified based on, for example, renal damage (albuminuria) or deterioration of estimated glomerular filtration rate (GFR < 60 [ml/min/1.73 m²], whether there is kidney damage).

In the present disclosure, a kidney disorder degree of a subject may be defined by the following estimated glomerular filtration rate (eGFR):
normal kidney function (CKD 1): eGFR ≥ 90 ml/min/1.73 m²
mild kidney function (CKD 2): 60 ml/min/1.73 m² ≤ eGFR < 90 ml/min/1.73 m2
moderate kidney function (CKD 3): 30 ml/min/1.73 m² ≤ eGFR < 60 ml/min/1.73 m²
severe kidney disorder (CKD 4): 15 ml/min/1.73 m² ≤ eGFR < 30 ml/min/1.73 m²
renal failure (CKD 5): eGFR < 15 ml/min/1.73 m²

Therefore, in the present disclosure, patients with a normal kidney function have the eGFR of 90 ml/min/1.73 m² or more, patients with mild kidney disorder have the eGFR of 60 ml/min/1.73 m² or more and less than 90 ml/min/1.73 m², patients with moderate kidney disorder have the eGFR of 30 ml/min/1.73 m² or more and less than 60 ml/min/1.73 m², patients with severe kidney disorder have the eGFR of 15 ml/min/1.73 m² or more and less than 30 ml/min/1.73 m², and patients with renal failure have the eGFR of less than 15 ml/min/1.73 m².

According to the present disclosure, the severe kidney disease may be classified into two subgroups:
severe kidney disorder A (CKD 2A): 75 ml/min/1.73 m² ≤ eGFR < 90 ml/min/1.73 m²
severe kidney disorder B (CKD 2B): 60 ml/min/1.73 m² ≤ eGFR < 75 ml/min/1.73 m²

Therefore, in the present disclosure, patients with moderate kidney disorder A have the eGFR of 75 ml/min/1.73 m² or more and less than 90 ml/min/1.73 m², and patients with moderate kidney disorder B have the eGFR of 60 ml/min/1.73 m² or more and less than 75 ml/min/1.73 m².

According to the present disclosure, the moderate kidney disorder may be classified into two subgroups:
moderate kidney disorder A (CKD 3A): 45 ml/min/1.73 m² ≤ eGFR < 60 ml/min/1.73 m²
moderate kidney disorder B (CKD 3B): 30 ml/min/1.73 m² ≤ eGFR < 45 ml/min/1.73 m²

Therefore, in the present disclosure, patients with moderate kidney disorder A have the eGFR of 45 ml/min/1.73 m² or more and less than 60 ml/min/1.73 m², and patients with moderate kidney disorder B have the eGFR of 30 ml/min/1.73 m² or more and less than 45 ml/min/1.73 m².

In the present disclosure, a subject may refer to a mammal including a human, and for example, the subject may be a human.

In the present disclosure, "treatment" refers to reverse, alleviation, inhibition of progression of, or prevention of a disease or illness, or one or more symptoms of the disease or illness, unless otherwise stated, and the term "treatment" used in the present disclosure refers to the act of treating when the "treatment" is defined as described above. Therefore, the composition and the method of the present disclosure may be used for, for example, chronic treatment as well as treatment as a therapeutic treatment for a certain period of time.

In the present specification, the term "prevention" may refer to prevention or delay of onset of disease, disorder, or illness.

The composition of the present disclosure may be formulated into various forms, for example, oral formulations such as liquids, suspensions, powders, granules, tablets, capsules, pills, extracts, emulsions, syrups, and aerosols, and injection of a sterile injection solution according to a common method for each intended use and then used, and may be orally administered or parenterally administered through various routes including intravenous, intraperitoneal, subcutaneous, intradermal, intramuscular, spinal, intrathecal, or rectal topical administration or injection, and a dosage ranges may vary depending on, for example, a patient's weight, age, sex, health status, diet, administration time, administration method, administration period or interval, excretion rate, and constitutional specificity, the nature of the preparation, and the severity of disease. For example, the composition of the present disclosure may be an oral dosage form in a tablet form. Otherwise, the composition of the present disclosure may be an intravenous injection form in a liquid form.

The term "tablet" in the present specification refers to a medicine compressed into a small disc shape for easy taking. The tablet may include, for example, an uncoated tablet, a film-coated tablet, a sugar-coated tab let, a multilayer tab let, a cored tablet, an inner-core tablet, an orally disintegrating tablet, a chewable tab let, an effervescent tablet, a dispersible tablet, and a dissolving tablet.

In one exemplary embodiment, the composition of the present disclosure may be a composition for oral administration. The term "oral administration" in the present specification may refer to administration of an active material to a gastrointestinal tract by an oral route for absorption. A non-limiting example of the preparation for oral administration may include, for example, tablets, troches, lozenges, aqueous suspensions, oily suspensions, prepared powders, granules, emulsions, hard capsules, soft capsules, syrups, or elixirs. In order to prepare the pharmaceutical composition of the present disclosure into a formulation for oral administration, a pharmaceutically acceptable additive or carrier may be further included. For example, the pharmaceutical composition of the present disclosure may use, for example, binders such as lactose, saccharose, sorbitol, mannitol, starch, amylopectin, cellulose, or gelatin; excipients such as dicalcium phosphate; disintegrants such as corn starch or sweet potato starch; and lubricants such as magnesium stearate, calcium stearate, sodium stearyl fumarate, or polyethylene glycol, and also, for example, sweeteners, flavorings, or syrups. Furthermore, the capsule may further use, for example, a liquid carrier such as fatty oil in addition to the materials mentioned above.

The composition of the present disclosure may be a composition for parenteral administration. The term "parenteral administration" in the present specification refers to intravenous, intraperitoneal, subcutaneous, intradermal, intramuscular, spine, intrathecal, or rectal topical administration or injection. The parenteral administration depends on a method of injecting suppository, subcutaneous injection, intramuscular injection, or intrathoracic injection. Herein, the composition is mixed with a stabilizer or buffer in water for preparing into a formulation for parenteral administration and prepared into a solution or suspension, which may be prepared into a unit dosage form of an ampoule or vial.

The pharmaceutical composition according to one exemplary embodiment of the present disclosure may further include other active ingredients in addition to the allopurinol, the febuxostat, or the pharmaceutically acceptable salt thereof. For example, the pharmaceutical composition according to one exemplary embodiment may further include other therapeutic agents for kidney disease. For example, the pharmaceutical composition according to one exemplary embodiment may further include one or two or more of active ingredients such as an angiotensin converting enzyme inhibitor (ACE I) or an angiotensin II receptor blocker (ARB). The pharmaceutical composition according to one exemplary embodiment of the present disclosure may be, for example, a composition for administration in combination.

In the present disclosure, the term "pharmaceutically acceptable salt" refers to a salt commonly used in the pharmaceutical industry. For example, it may refer to an acid addition salt formed by a pharmaceutically acceptable free acid. For example, a non-limiting example of the pharmaceutically acceptable salt may include inorganic ion salts prepared by, for example, calcium, potassium, sodium, or magnesium, or inorganic acid salts prepared by, for example, hydrochloric acid, nitric acid, bromic acid, iodic acid, perchloric acid, or sulfuric acid; organic acid salts prepared by, for example, acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, or vanillic acid; sulfonic acid salts prepared by, for example, methanesulfonic acid, ethanesulfonic acid, benzenesulfonic acid, p-toluenesulfonic acid, or naphthalene sulfonic acid; amino acid salts prepared by glycine, arginine, or lysine; or amine salts prepared by, for example, trimethylamine, triethyleamine, ammonia, pyridine, or picoline.

According to one exemplary embodiment of the present disclosure, a pharmaceutical composition for use in preventing or treating chronic kidney disease of a subject having a serum uric acid concentration of 6 mg/dl or more, including a therapeutically effective amount of allopurinol, febuxostat, or a pharmaceutically acceptable salt thereof as an active ingredient may be provided.

In one exemplary embodiment, the subject may have a blood glucose level of 126 mg/dl or more, a diastolic blood pressure of 80 mmHg or lower, and/or a systolic blood pressure of 130 mmHg or lower. The subject may have the blood glucose level of 126 mg/dl or more. The subject may have the diastolic blood pressure of 80 mmHg or lower. The subject may have the systolic blood pressure of 130 mmHg or lower. The subject may have the diastolic blood pressure of 80 mmHg or lower and the systolic blood pressure of 130 mmHg or lower.

In one exemplary embodiment, the active ingredient may be the allopurinol or the pharmaceutically acceptable salt thereof, and the subject may not have congestive cardiac failure.

In one exemplary embodiment, the active ingredient may be the febuxostat or the pharmaceutically acceptable salt thereof, and the subject may have the serum uric acid concentration of 8 mg/dl or more.

In one exemplary embodiment, the subject may have a serum uric acid concentration of 7 mg/dl or more. The subject may be a patient with hyperuricacidemia or gout who has the serum uric acid concentration of 7 mg/dl or more.

In one exemplary embodiment, the subject may have hyperuricacidemia or gout. In one exemplary embodiment, the subject may have gout. In one exemplary embodiment, the active ingredient may be the febuxostat or the pharmaceutically acceptable salt thereof, and the gout may have lasted 10 years or more.

In one exemplary embodiment, the subject may not have hypertension. In one exemplary embodiment, the subject may have normal or low blood pressure.

Since the subject having hypertension has increased force of blood pushing against a blood vessel wall, the blood vessel wall is prone to tension or damage and kidney blood vessels may be thickened or hardened. Therefore, blood is not supplied well to the kidneys and kidney function may be gradually declined. When loss of kidney function becomes severe, body waste is not removed and accumulates in the body, and when moisture excessively accumulates, a vicious cycle occurs in which blood pressure becomes more poorly controlled. When this is left untreated, it leads eventually to end-stage renal failure in which the kidney function is almost lost. That is, it is known that the chronic kidney disease is usually more likely to occur and more dangerous in hypertension patients.

As described in more detail later, the pharmaceutical composition according to one exemplary embodiment was confirmed to show a better effect when it is administered to a subject having a diastolic blood pressure of 80 mmHg or lower and/or a systolic blood pressure of 130 mmHg or lower. That is, the pharmaceutical composition according to one exemplary embodiment is more effective in the subject having a diastolic blood pressure of 80 mmHg or lower and/or a systolic blood pressure of 130 mmHg or lower and a serum uric acid concentration of 6 mg dl or 7 mg/dl or more.

In general, considering that a subject with hypertension has a higher risk of developing chronic kidney disease, the effect of the present disclosure is more surprising as the present disclosure exhibits better efficacy in a patient having a blood pressure range of a diastolic blood pressure of 80 mmHg or lower and/or a systolic blood pressure of 130 mmHg or lower.

In one exemplary embodiment, the composition may decrease the serum uric acid concentration of the subject to less than 6 mg/dl. The composition of one exemplary embodiment maintains the serum uric acid concentration of the subject at less than 6 mg/dl to prevent or treat the chronic kidney disease. In particular, the pharmaceutical composition of one exemplary embodiment may maintain the eGFR of the subject or slow down the rate of decline to show an excellent effect in both prevention and treatment of the chronic kidney disease.

In one exemplary embodiment, the active ingredient may be the allopurinol or the pharmaceutically acceptable salt thereof, and a dosage of the active ingredient may be 100 mg to 600 mg, 300 mg to 600 mg, or 200 mg to 400 mg per day, and for example, 100 mg, 200 mg, 300 mg, 400 mg, 500 mg, or 600 mg per day, based on the amount of the allopurinol.

In one exemplary embodiment, the active ingredient may be the febuxostat or the pharmaceutically acceptable salt thereof, and the dosage of the active ingredient may be 20 mg to 120 mg, 20 mg to 100 mg, or 40 mg to 80 mg per day, for example, 20 mg, 40 mg, 60 mg, 80 mg, 100 mg, or 120 mg per day, based on the amount of the febuxostat.

The daily dosage in the present disclosure may be a dosage administered 1 to 5 times per day, for example, a dosage administered once a day, twice a day, or 3 times a day, and in particular, a dosage administered once a day.

Unless otherwise mentioned in the present disclosure, the dosage or content of the pharmaceutically acceptable salt of the active ingredient may be based on the amount of the active ingredient excluding salts. That is, when it is mentioned in the present specification that the dosage or content of the pharmaceutically acceptable salt of the allopurinol is 200 mg, it is interpreted as meaning 200 mg of allopurinol excluding salts.

In one exemplary embodiment, the eGFR of the subject may be 45 ml/min/1.73 m² or more. The pharmaceutical composition of one exemplary embodiment may show an excellent effect in the subject with chronic kidney disease at stages of CKD 1 to CKD 3A. That is, the pharmaceutical composition of one exemplary embodiment may be administered to a subject having the eGFR of 45 ml/min/1.73 m² or more to show an excellent effect.

In one exemplary embodiment, the active ingredient may be the allopurinol or the pharmaceutically acceptable salt thereof. The allopurinol or the pharmaceutically acceptable salt may be administered to a subject having the eGFR of 60 ml/min/1.73 m² or more at a dose of 300 mg per day, and if the serum uric acid concentration of the subject is not decreased to less than 6 mg/dl at one month after the administration, the active ingredient may be administered at a dose of 600 mg per day.

The allopurinol or the pharmaceutically acceptable salt thereof may be administered to a subject having the eGFR of 30 ml/min/1.73 m² or more and less than 60 mg/min/1.73 m² at a dose of 200 mg per day, and if the serum uric acid concentration of the subject is not decreased to less than 6 mg/dl at one month after the administration, the active ingredient may be administered at a dose which increased in increments of 100 mg per month up to a maximum of 400 mg per day.

In one exemplary embodiment, the active ingredient may be the febuxostat or the pharmaceutically acceptable salt thereof. The febuxostat or the pharmaceutically acceptable salt thereof may be administered at a dose of 40 mg per day based on the amount of the febuxostat, and if the serum uric acid concentration of the subject is not decreased to less than 6 mg/dl at 2 weeks after the administration, the active ingredient may be administered at a dose of 80 mg per day based on the amount of the febuxostat.

The pharmaceutical composition of one exemplary embodiment may be administered to a patient with a major cardiovascular disease history. The major cardiovascular disease history may be at least one selected from myocardial infarction, hospitalization for unstable angina, stroke, hospitalization for transient ischemic attack, peripheral vascular disease and diabetes mellitus symptoms.

The pharmaceutical composition of one exemplary embodiment may be a composition in an oral dosage form. The pharmaceutical composition of one exemplary embodiment may be a tablet in an oral dosage form. The pharmaceutical composition of one exemplary embodiment may be an intravenous dosage form.

Hereinafter, the present disclosure will be described in more detail based on the following examples. These examples are provided merely to illustrate the present disclosure, and it will be apparent to a person skilled in the art that the scope of the present disclosure is not construed to be limited to the examples.

### Examples

### Design of clinical trial

In order to evaluate non-inferiority of febuxostat to allopurinol related to cardiovascular risk, a clinical trial was designed and proceeded. An effect of a pharmaceutical composition including allopurinol, febuxostat, or a pharmaceutically acceptable salt thereof according to one exemplary embodiment on kidney disease was derived from the data derived from the clinical trial.

### Trial design

A multicenter, randomized, double-blind non-inferiority trial was performed. The details of the clinical trial design have been published previously. An independent data and safety monitoring committee with access to unblinded data monitored the clinical trial. The statistical analysis for the data and the safety monitoring committee was performed by an independent statistical group (WebbWrites).

### Patient group

Patients who were diagnosed with gout fulfilling category 9 of the standards set by the American Rheumatism Association had a history of major cardiovascular diseases before randomization were enrolled as participants in the clinical trial. As an additional criterion for clinical trial participation, patients with inappropriately controlled gout who had a serum uric acid concentration of at least 7.0 mg per deciliter (420 µmol per liter) or more or 6.0 mg per deciliter (360 µmol) or more after a 1 to 3 weeks washout period from the previous gout therapies were required. The patients were regarded as having a history of major cardiovascular diseases, if they had myocardial infarction, hospitalization for unstable angina, stroke, hospitalization for transient ischemic attack, peripheral vascular disease, or diabetes mellitus symptoms. All participants provided a prior informed consent form.

### Treatments and procedures

The clinical trial participants participated in the clinical trial according to the following procedures. Hereinafter, unless otherwise particularly mentioned, a dose of a drug was described based on an amount of allopurinol or febuxostat.

### (1) Allopurinol administration group

The patients were randomly assigned so that they received febuxostat or allopurinol administered once a day in a double-blind manner. The randomization was stratified according to the estimated creatinine clearance at baseline (patient group having an estimated creatinine clearance ≥60 ml per minute vs patient group having an estimated creatinine clearance ≥30 and <60 ml per minute).

The dose of allopurinol was modified according to a kidney function. The patients with the estimated creatinine clearance 60 ml or more per minute i) initially received allopurinol at a dose of 300 mg once a day, and the dose was increased in 100 mg increments monthly until the patient had the serum uric acid concentration of less than 6.0 mg per deciliter or the dosage of allopurinol was 600 mg once a day. The patient having the estimated creatinine clearance of 30 ml or more and less than 60 ml per minute initially received 200 mg of allopurinol, and then the dose was increased in 100 mg increments until the patient either had the serum uric acid concentration of less than 6.0 mg per deciliter or was receiving the allopurinol dose of 400 mg once daily.

### (2) Febuxostat administration group

The dose of febuxostat was not modified according to the kidney function. The patients who were randomly assigned to receive febuxostat initially received 40 mg once a daily, and continued to receive the same dosage if the serum uric acid concentration was less than 6.0 mg per deciliter after 2 weeks of the treatment. If the serum uric acid concentration was higher than 6.0 mg per deciliter at 2nd week visit, the dose of febuxostat was increased to 80 mg once daily for the remainder of the trial.

### (3) Procedure

The serum uric acid concentrations of the patients were revealed to the site investigators only during the dose adjustment period of 10 weeks to facilitate dose increases based on a uric acid response. During the period, the administration of a double-blind and double-dummy trial medications was guided by an interactive voice-response system which was a procedure to prevent unblinding for the patients whose dose was not adjusted. After completing dose adjustment, the urate level was concealed from the investigators, and the treatment was managed throughout the trial using the interactive voice-response system.

At the screening visit, all uric acid lowering therapy was discontinued, and a colchicine treatment at a dose of 0.6 mg per day was started for preventing a gout flare unless the patient had a history of unacceptable side effects due to colchicine. All patients received prophylaxis for the first 6 months of the randomly assigned treatment. If the colchicine treatment resulted in unacceptable side effects and the estimated creatinine clearance was at least 50 ml per minute, the patient received naproxen (250 mg, twice a day) with lansoprazole (15 mg, once a day). If the patient did not receive colchicine or naproxen, other nonsteroidal anti-inflammatory drugs (NSAID) or prednisone could be provided as prophylaxis, or the investigators could choose to manage a gout flare, if it occurred.

Outpatient visits were conducted according to the following schedule: during screening and randomization, and at 2, 4, 6, 8, 10, 12, and 24 weeks after the randomization, and every 6 months during the subsequent years of the trial. The patients with reduced kidney function or, the patients aged 65 years or older at randomization were visited at 9 and 15 months for monitoring the serum chemical profile. If the patient agreed to be monitored, but would not return for study visit, telephone contact was made, but such method was not preferred or recommended.

### End point

A first occurrence of cardiovascular death, nonfatal myocardial infarction, nonfatal stroke, or urgent revascularization for unstable angina were set as a primary composite end point.

A secondary safety end point included a composite of cardiovascular death, nonfatal myocardial infarction, and nonfatal stroke, and the individual components of the primary evaluation variables. The consistency of the effects on the primary end point was investigated in various subgroups (both pre-specified and post-hoc designation). An additional safety end point included death from all causes, urgent cerebral revascularization, transient ischemic attack, hospitalization for cardiac failure, arrhythmias not associated with ischemia, and venous thromboembolic events. An independent central end point committee consisting of members who did not know about the treatment assignment adjudicated all suspected end point events.

### Results

### Patient group

6198 patients from 320 locations across North America enrolled from April 2010 to May 2017. 8 patients did not receive the trial medication, and 6190 patients received modified intention to treat analysis. Two treatment groups were confirmed to be well balanced with regard to all baseline characteristics. In a febuxostat group, 61.0% of the patients received 40 mg of febuxostat every day, and 39.0% of the patients received 80 mg of febuxostat every day as a final adjusted dose. In an allopurinol group, 21.8% of the patients received 200 mg of allopurinol, 44.6% of the patients received 300 mg of allopurinol, 25.2% of the patients received 400 mg of allopurinol, 4.3% of the patients received 500 mg of allopurinol, and 4.1% of the patients received 600 mg of allopurinol, on the basis of the protocol-directed criteria for the estimated creatinine clearance.

Overall, 56.6% of the patients discontinued the trial treatment prematurely. The early discontinuation rates were similar in the febuxostat and allopurinol groups (57.3% in the febuxostat group and 55.9% in the allopurinol group). A percentage of the patients who did not complete all trial visits was 45.0% (45.0% in the febuxostat, 44.9% in the allopurinol group). The median duration of exposure to febuxostat was 728 days, and the median duration of exposure to allopurinol was 719 days. The median duration of a follow-up period was 968 days in the febuxostat group and 942 days in the allopurinol group.

### Biochemical effect

A proportion of the patients with the serum uric acid concentration of less than 6.0 mg per deciliter was higher in the febuxostat group than in the allopurinol group at the 2nd week. Thereafter, though a difference between the groups was not large, a higher proportion of the patients in the febuxostat group had maintenance of the serum uric acid concentration at less than 6.0 mg per deciliter at most times. In addition, a larger proportion of the patients in the febuxostat group than in the allopurinol group had the serum uric acid concentration of less than 5.0 mg per deciliter (300 µmol per liter) for the entire trial. Overall, the rates of the gout flare were similar in the two treatment groups (flares of 0.68 and 0.63, per patient-year in the febuxostat group and the allopurinol group, respectively). During the trial period, there was no significant difference in serum levels of electrolyte, glucose, or lipids, or blood pressure, and also, there was no difference in use of cardiovascular medications.

### Analysis of effect on kidney disease

In order to confirm the effect of allopurinol and febuxostat on the kidney disease, the data obtained in the clinical trial described above was analyzed.

The patients were classified into patient groups by specific biomarkers such as blood glucose level, blood pressure, and kidney disease steps, and then risks of developing or worsening the kidney disease in patients who had the serum uric acid concentration decreased to less than 6 and patients who maintained the serum uric acid concentration at or above 6 were confirmed using odds ratios (OR) after the administration of allopurinol and febuxostat, and the results are shown in Tables 1 to 3.

In Tables 1 to 3, a confidence interval is indicated as CI, systolic blood pressure is indicated as SBP, and diastolic blood pressure is indicated as DBP.

Risks of developing the kidney disease of the patients who had the serum uric acid concentration decreased to less than 6 and the patients who maintained the serum uric acid concentration at or above 6 by administering allopurinol were confirmed using the odds ratios, and the results are shown in the following Table 1.

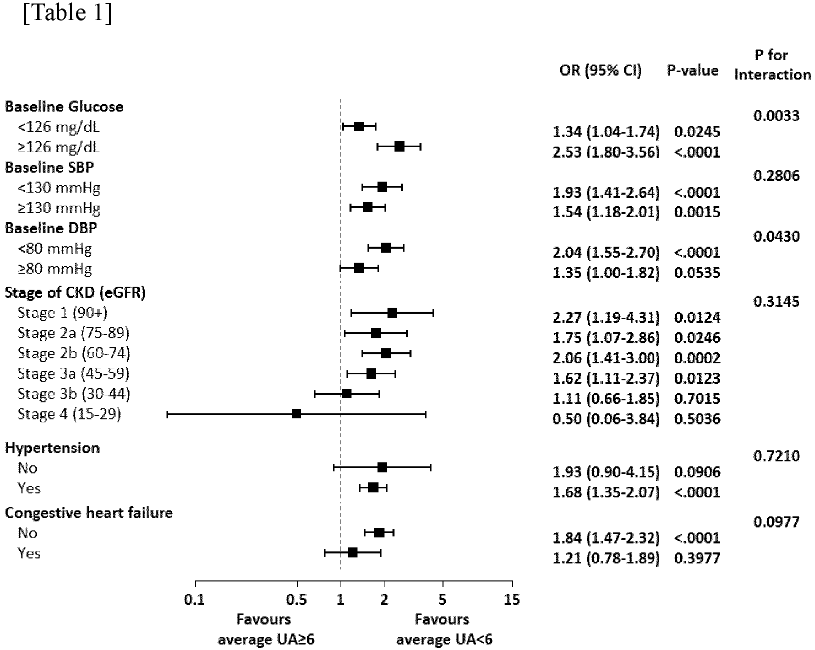

Referring to Table 1, in the patient group having the blood glucose level of 126 mg/dl or less, if the serum uric acid concentration was maintained at or above 6, the risk of developing or worsening the chronic kidney disease was increased by about 1.34 times as compared with the case in which the serum uric acid concentration was maintained below 6, but in the patient group having the blood glucose level of 126 mg/dl or more, if the serum uric acid concentration was maintained at or above 6, the risk of developing or worsening the chronic kidney disease was increased by about 2.53 times as compared with the case in which the serum uric acid concentration was maintained below 6. In addition, in the patient group having the diastolic blood pressure of 80 mm/Hg or higher, if the serum uric acid concentration was maintained at or above 6, the risk of developing or worsening the kidney disease was increased by about 1.35 times as compared with the case in which the serum uric acid concentration was maintained below 6, but in the patient group having the diastolic blood pressure of 80 mm/Hg or lower, if the serum uric acid concentration was maintained at or above 6, the risk of developing or worsening the chronic kidney disease was increased by about 2.04 times as compared with the case in which the serum uric acid concentration was maintained below 6. In addition, in the patient group having the systolic blood pressure of 130 mm/Hg or higher, if the serum uric acid concentration was maintained at or above 6, the risk of developing or worsening the chronic kidney disease was increased by about 1.54 times as compared with the case in which the serum uric acid concentration was maintained below 6, but in the patient group having the systolic blood pressure of 130 mm/Hg or lower, if the serum uric acid concentration was maintained at or above 6, the risk of developing or worsening the chronic kidney disease was increased by about 1.93 times as compared with the case in which the serum uric acid concentration was maintained below 6. In addition, in the patient group having no congestive cardiac failure, if the serum uric acid concentration was maintained at 6 or more, the risk of developing or worsening the chronic kidney disease was increased by about 1.84 times as compared with the case in which the serum uric acid concentration was maintained below 6, but in the patient group having congestive cardiac failure, if the serum uric acid concentration was maintained at 6 or more, the risk of developing or worsening the chronic kidney disease was increased by about 1.21 times as compared with the case in which the serum uric acid concentration was maintained below 6.

In addition, it was confirmed that the pharmaceutical composition according to one exemplary embodiment was more effective for the patients with the chronic kidney disease that is equal to or less severe than the moderate A chronic kidney disease (CKD 3A) with the eGFR of 45 ml/min/1.73 m² or more, and it was also more effective for the patients without hypertension.

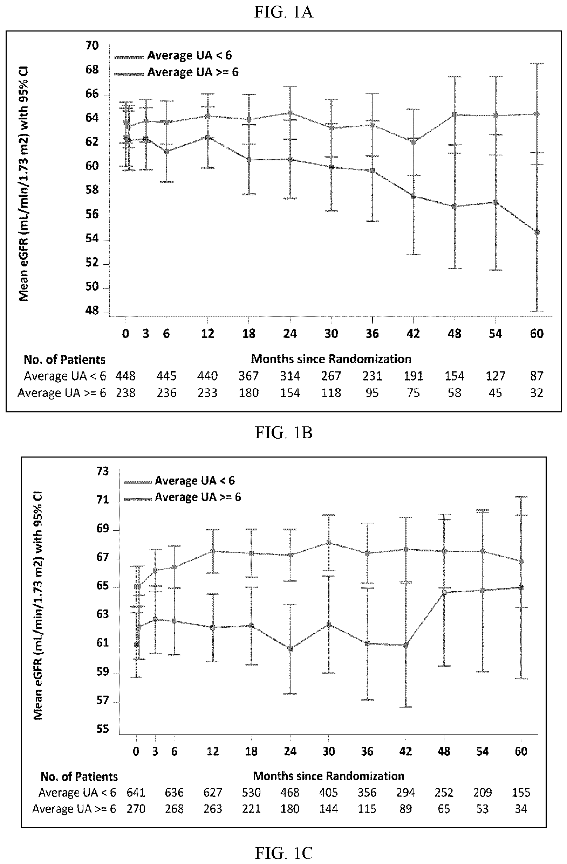

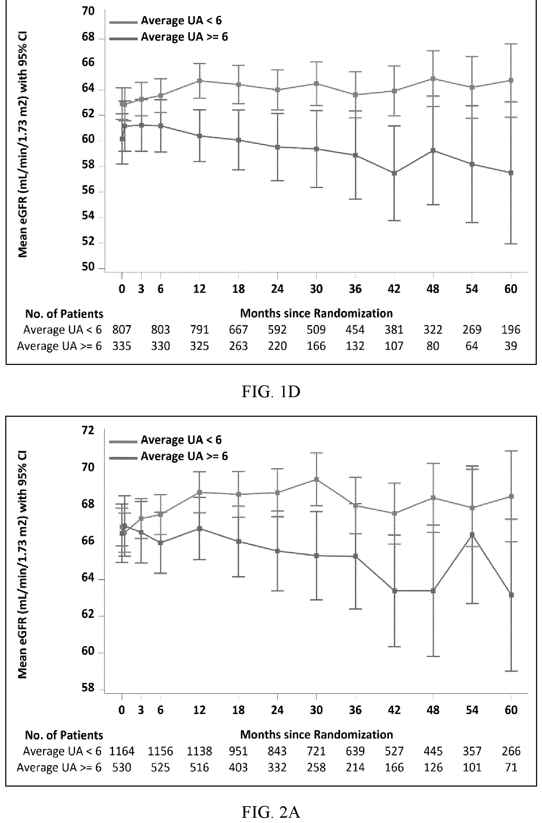

Specific data is described in Tables 2 to 5. In Table 2, the change in eGFR over time for each patient group classified depending on the serum uric acid concentration in the allopurinol administration group having a blood glucose level of 126 mg/dl or higher is described, and is graphed in FIG. 1A. In Table 3, the change in eGFR over time for each patient group classified depending on the serum uric acid concentration in the allopurinol administration group having a systolic blood pressure of 130 mm/hg or lower is described, and is graphed in FIG. 1B. In Table 4, the change in eGFR over time for each patient group classified depending on the serum uric acid concentration in the allopurinol administration group having a diastolic blood pressure of 80 mm/hg or lower is described, and is graphed in FIG. 1C. In Table 5, the change in eGFR over time for each patient group classified depending on the serum uric acid concentration in the allopurinol administration group having no congestive cardiac failure history is described, and is graphed in FIG. 1D.

In Tables 2 to 5, all p-values were calculated by a linear mixed model using a randomized intercept model for repeated measurement analysis, and the p values for the interaction effect for the eGFR value and the average serum uric acid concentration for each visit were all 0.0001 or less.

**[Table 2]**

| Baseline blood glucose level ≥ 126 | Average UA < 6 | | Average UA ≥ 6 | |
|---|---|---|---|---|
| | N | Mean (95% CI) | N | Mean (95% CI) |
| Baseline | 448 | 63.77 (62.08-65.46) | 238 | 62.56 (60.14-64.98) |
| Week 2 | 445 | 63.46 (61.70-65.22) | 236 | 62.27 (59.80-64.73) |
| Month 3 | 442 | 63.92 (62.16-65.69) | 237 | 62.44 (59.88-65.00) |
| Month 6 | 445 | 63.77 (61.98-65.56) | 236 | 61.37 (58.85-63.90) |
| Month 12 | 440 | 64.33 (62.51-66.15) | 233 | 62.57 (60.02-65.12) |
| Month 18 | 367 | 64.04 (61.97-66.10) | 180 | 60.70 (57.80-63.60) |
| Month 24 | 314 | 64.60 (62.41-66.78) | 154 | 60.72 (57.46-63.99) |
| Month 30 | 267 | 63.32 (60.93-65.71) | 118 | 60.06 (56.44-63.69) |
| Month 36 | 231 | 63.57 (60.97-66.18) | 95 | 59.78 (55.61-63.95) |
| Month 42 | 191 | 62.14 (59.40-64.89) | 75 | 57.66 (52.84-62.48) |
| Month 48 | 154 | 64.42 (61.23-67.61) | 58 | 56.80 (51.66-61.93) |
| Month 54 | 127 | 64.35 (61.09-67.61) | 45 | 57.15 (51.53-62.78) |
| Month 60 | 87 | 64.49 (60.29-68.70) | 32 | 54.70 (48.12-61.28) |

**[Table 3]**

| SBP < 130 | Average UA < 6 | | Average UA ≥ 6 | |
|---|---|---|---|---|
| | N | Mean (95% CI) | N | Mean (95% CI) |
| Baseline | 641 | 65.08 (63.67-66.50) | 270 | 61.00 (58.76-63.25) |
| Week 2 | 640 | 65.12 (63.70-66.55) | 269 | 62.24 (60.01-64.47) |
| Month 3 | 640 | 66.19 (64.73-67.66) | 267 | 62.77 (60.42-65.12) |
| Month 6 | 636 | 66.44 (64.96-67.92) | 268 | 62.65 (60.33-64.97) |
| Month 12 | 627 | 67.55 (66.04-69.06) | 263 | 62.21 (59.86-64.55) |
| Month 18 | 530 | 67.41 (65.73-69.08) | 221 | 62.34 (59.64-65.04) |
| Month 24 | 468 | 67.26 (65.46-69.07) | 180 | 60.72 (57.61-63.83) |
| Month 30 | 405 | 68.14 (66.20-70.08) | 144 | 62.43 (59.06-65.81) |
| Month 36 | 356 | 67.41 (65.30-69.51) | 115 | 61.08 (57.19-64.97) |
| Month 42 | 294 | 67.67 (65.45-69.89) | 89 | 60.98 (56.66-65.31) |
| Month 48 | 252 | 67.55 (64.98-70.13) | 65 | 64.66 (59.55-69.76) |
| Month 54 | 209 | 67.54 (64.81-70.27) | 53 | 64.80 (59.14-70.46) |
| Month 60 | 155 | 66.85 (63.63-70.06) | 34 | 65.01 (58.65-71.37) |

**[Table 4]**

| DBP < 80 | Average UA < 6 | | Average UA ≥ 6 | |
|---|---|---|---|---|
| | N | Mean (95% CI) | N | Mean (95% CI) |
| Baseline | 807 | 62.91 (61.68-64.15) | 335 | 60.15 (58.18-62.12) |
| Week 2 | 803 | 62.85 (61.57-64.14) | 332 | 61.16 (59.19-63.13) |
| Month 3 | 803 | 63.26 (61.95-64.58) | 331 | 61.22 (59.20-63.24) |
| Month 6 | 803 | 63.55 (62.23-64.87) | 330 | 61.19 (59.14-63.24) |
| Month 12 | 791 | 64.71 (63.34-66.07) | 325 | 60.39 (58.37-62.42) |
| Month 18 | 667 | 64.41 (62.91-65.90) | 263 | 60.06 (57.73-62.40) |
| Month 24 | 592 | 64.00 (62.43-65.57) | 220 | 59.51 (56.88-62.15) |
| Month 30 | 509 | 64.49 (62.77-66.21) | 166 | 59.37 (56.35-62.39) |
| Month 36 | 454 | 63.61 (61.80-65.43) | 132 | 58.89 (55.43-62.34) |
| Month 42 | 381 | 63.91 (61.95-65.87) | 107 | 57.48 (53.76-61.19) |
| Month 48 | 322 | 64.88 (62.69-67.07) | 80 | 59.25 (55.00-63.51) |
| Month 54 | 269 | 64.19 (61.76-66.62) | 64 | 58.18 (53.60-62.76) |
| Month 60 | 196 | 64.73 (61.86-67.61) | 39 | 57.50 51.94-63.07) |

**[Table 5]**

| No congestive cardiac failure history | Average UA < 6 | | Average UA ≥ 6 | |
|---|---|---|---|---|
| | N | Mean (95% CI) | N | Mean (95% CI) |
| Baseline | 1164 | 66.85 (65.82-67.88) | 530 | 66.51 (64.92-68.09) |
| Week 2 | 1158 | 66.55 (65.48-67.62) | 525 | 66.92 (65.28-68.56) |
| Month 3 | 1158 | 67.32 (66.24-68.40) | 525 | 66.56 (64.90-68.23) |
| Month 6 | 1156 | 67.53 (66.44-68.62) | 525 | 65.99 (64.33-67.66) |
| Month 12 | 1138 | 68.74 (67.63-69.86) | 516 | 66.77 (65.07-68.46) |
| Month 18 | 951 | 68.64 (67.39-69.88) | 403 | 66.07 (64.15-67.99) |
| Month 24 | 843 | 68.73 (67.42-70.03) | 332 | 65.54 (63.37-67.72) |
| Month 30 | 721 | 69.45 (68.01-70.89) | 258 | 65.30 (62.89-67.70) |
| Month 36 | 639 | 68.02 (66.49-69.55) | 214 | 65.27 (62.40-68.13) |
| Month 42 | 527 | 67.59 (65.93-69.25) | 166 | 63.38 (60.35-66.41) |
| Month 48 | 445 | 68.44 (66.56-70.31) | 126 | 63.38 (59.80-66.95) |
| Month 54 | 357 | 67.90 (65.79-70.00) | 101 | 66.44 (62.69-70.18) |
| Month 60 | 266 | 68.53 (66.05-71.01) | 71 | 63.14 (59.01-67.28) |

Referring to Tables 2 to 5 and FIGS. 1A to 1D, if each patient group classified based on the four biomarkers maintained the serum uric acid concentration below 6, it was confirmed that the eGFR was maintained or rather increased. However, when the serum uric acid concentration was 6 or more, it was confirmed that the eGFR was decreased.

That is, it was confirmed that if allopurinol is administered to a patient group having the blood glucose level of 126 mg/dl or higher, the diastolic blood pressure of 80 mm/Hg or lower, the systolic blood pressure of 130 mm/Hg or lower, or no congestive cardiac failure to maintain the serum uric acid concentration below 6, the chronic kidney disease may be effectively prevented or treated.

In Table 6, the risks of developing the kidney disease of the patients who had the serum uric acid concentration decreased to less than 6 and the patients who maintained the serum uric acid concentration at or above 6 by administering febuxostat are confirmed using the odds ratios.

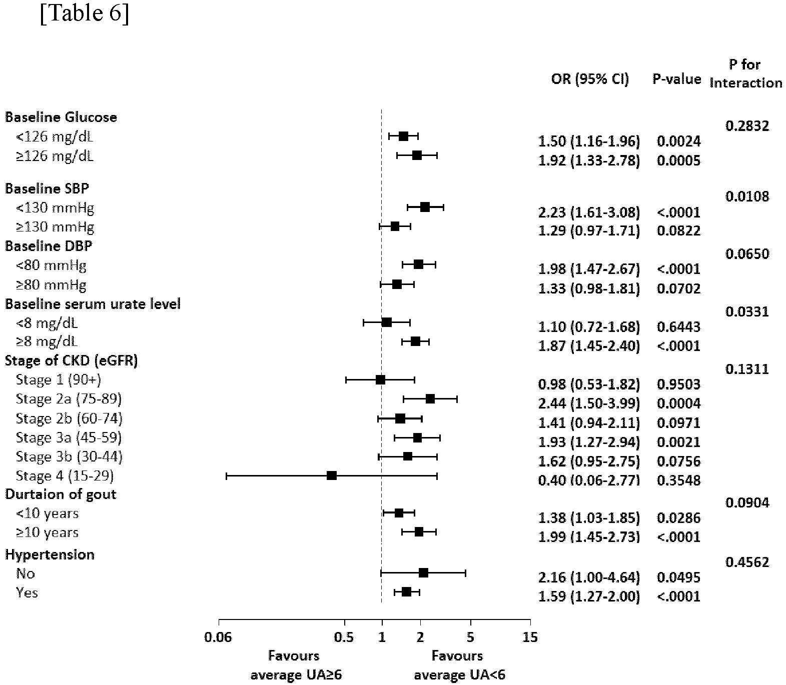

Referring to Table 6, as with the results when allopurinol was administered, it was confirmed that if febuxostat is administered to a patient group having a blood glucose level of 126 mg/dl or more, a diastolic blood pressure of 80 mm/Hg or lower, or a systolic blood pressure or 130 mm/Hg or lower to maintain the serum uric acid concentration below 6, the chronic kidney disease may be effectively prevented or treated. In addition, it was confirmed that if febuxostat is administered to a patent group having the serum uric acid concentration of 8 mg/dL or more or gout lasting for 10 years or more to maintain the serum uric acid concentration below 6, the chronic kidney disease may be effectively prevented or treated.

In addition, it was confirmed that the pharmaceutical composition according to one exemplary embodiment was more effective for the patients with the chronic kidney disease that is equal to or less severe than the moderate A chronic kidney disease (CKD 3A) with the eGFR of 45 ml/min/1.73 m² or more, and it was also more effective for the patients without hypertension.

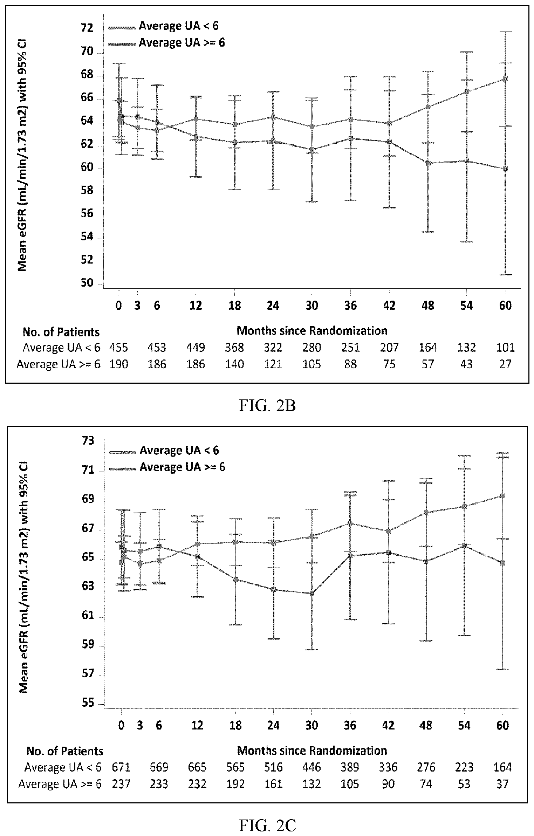

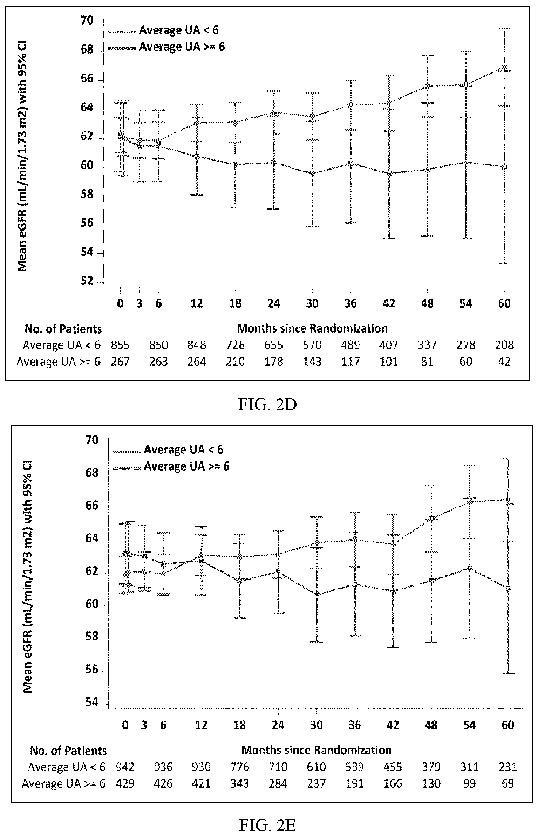

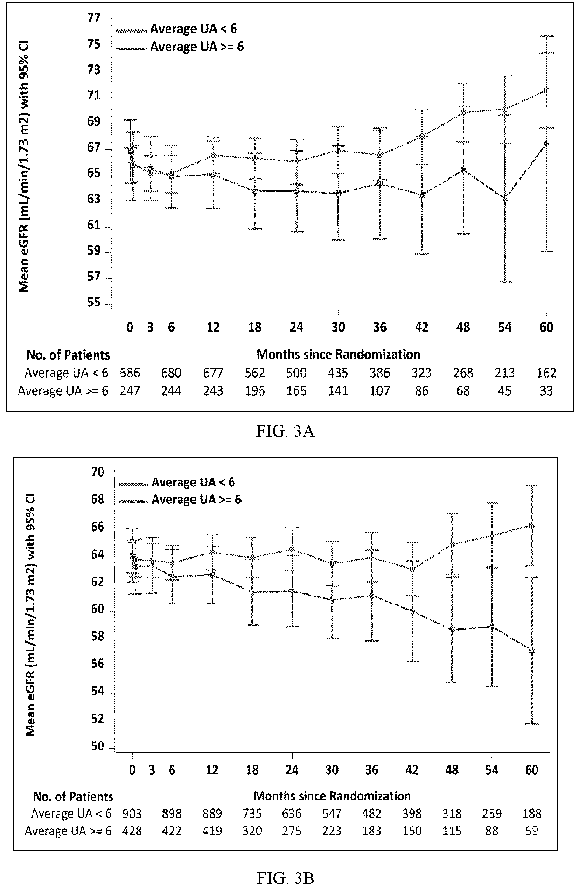

Specific data is described in Tables 7 to 11. In Table 7, the change in eGFR over time for each patient group classified depending on the serum uric acid concentration in the febuxostat administration group having a blood glucose level of 126 mg/dl or higher is described, and is graphed in FIG. 2A. In Table 8, the change in eGFR over time for each patient group classified depending on the serum uric acid concentration in the febuxostat administration group having a systolic blood pressure of 130 mm/hg or lower is described, and is graphed in FIG. 2B. In Table 9, the change in eGFR over time for each patient group classified depending on the serum uric acid concentration in the febuxostat administration group having a diastolic blood pressure of 80 mm/hg or lower is described, and is graphed in FIG. 2C. In Table 10, the change in eGFR over time for each patient group classified depending on the serum uric acid concentration in the febuxostat administration group having a baseline serum uric acid concentration of 8 mg/dl or more is described, and is graphed in FIG. 2D. In Table 11, the change in eGFR over time for each patient group classified depending on the serum uric acid concentration in the febuxostat administration group having a gout history for 10 years or more is described, and is graphed in FIG. 2E.

In Tables 7 to 11, all p-values were calculated by a linear mixed model using a randomized intercept model for repeated measurement analysis, and the p values for the interaction effect for the eGFR value and the average serum uric acid concentration for each visit were all 0.0001 or less.

**[Table 7]**

| Baseline blood glucose level ≥ 126 | Average UA < 6 | | Average UA ≥ 6 | |
|---|---|---|---|---|
| | N | Mean (95% CI) | N | Mean (95% CI) |
| Baseline | 455 | 64.23 (62.54-65.93) | 190 | 65.94 (62.79-69.10) |
| Week 2 | 451 | 64.06 (62.29-65.83) | 186 | 64.56 (61.25-67.87) |
| Month 3 | 453 | 63.54 (61.75-65.32) | 187 | 64.51 (61.21-67.81) |
| Month 6 | 453 | 63.33 (61.52-65.14) | 186 | 64.04 (60.85-67.23) |
| Month 12 | 449 | 64.32 (62.49-66.15) | 186 | 62.81 (59.35-66.28) |
| Month 18 | 368 | 63.84 (61.79-65.89) | 140 | 62.28 (58.22-66.35) |
| Month 24 | 322 | 64.49 (62.28-66.69) | 121 | 62.44 (58.21-66.68) |
| Month 30 | 280 | 63.64 (61.38-65.91) | 105 | 61.66 (57.17-66.16) |
| Month 36 | 251 | 64.30 (61.77-66.83) | 88 | 62.64 (57.30-67.99) |
| Month 42 | 207 | 63.94 (61.12-66.76) | 75 | 62.33 (56.68-67.99) |
| Month 48 | 164 | 65.35 (62.27-68.43) | 57 | 60.51 (54.59-66.44) |
| Month 54 | 132 | 66.66 (63.21-70.10) | 43 | 60.70 (53.73-67.67) |
| Month 60 | 101 | 67.78 (63.68-71.89) | 27 | 60.00 (50.87-69.14) |

**[Table 8]**

| SBP < 130 | Average UA < 6 | | Average UA ≥ 6 | |
|---|---|---|---|---|
| | N | Mean (95% CI) | N | Mean (95% CI) |
| Baseline | 671 | 64.76 (63.32-66.20) | 237 | 65.83 (63.23-68.44) |
| Week 2 | 669 | 65.16 (63.69-66.63) | 235 | 65.59 (62.82-68.36) |
| Month 3 | 667 | 64.67 (63.21-66.12) | 235 | 65.54 (62.88-68.20) |
| Month 6 | 669 | 64.87 (63.38-66.36) | 233 | 65.87 (63.30-68.43) |
| Month 12 | 665 | 66.06 (64.55-67.57) | 232 | 65.19 (62.39-67.99) |
| Month 18 | 565 | 66.18 (64.56-67.79) | 192 | 63.60 (60.49-66.71) |
| Month 24 | 516 | 66.12 (64.42-67.83) | 161 | 62.90 (59.50-66.30) |
| Month 30 | 446 | 66.59 (64.74-68.44) | 132 | 62.62 (58.76-66.48) |
| Month 36 | 389 | 67.47 (65.55-69.39) | 105 | 65.24 (60.84-69.64) |
| Month 42 | 336 | 66.93 (64.77-69.08) | 90 | 65.47 (60.56-70.39) |
| Month 48 | 276 | 68.21 (65.88-70.54) | 74 | 64.82 (59.40-70.23) |
| Month 54 | 223 | 68.62 (66.03-71.22) | 53 | 65.92 (59.74-72.11) |
| Month 60 | 164 | 69.36 (66.42-72.30) | 37 | 64.71 (57.42-72.01) |

**[Table 9]**

| DBP < 80 | Average UA < 6 | | Average UA ≥ 6 | |
|---|---|---|---|---|
| | N | Mean (95% CI) | N | Mean (95% CI) |
| Baseline | 855 | 62.24 (61.02-63.45) | 267 | 62.08 (59.70-64.45) |
| Week 2 | 849 | 62.06 (60.81-63.31) | 263 | 62.00 (59.39-64.62) |
| Month 3 | 849 | 61.85 (60.63-63.08) | 264 | 61.44 (58.99-63.89) |
| Month 6 | 850 | 61.84 (60.56-63.11) | 263 | 61.49 (59.02-63.95) |
| Month 12 | 848 | 63.06 (61.79-64.33) | 264 | 60.74 (58.06-63.41) |
| Month 18 | 726 | 63.11 (61.75-64.47) | 210 | 60.18 (57.20-63.15) |
| Month 24 | 655 | 63.79 (62.31-65.27) | 178 | 60.32 (57.11-63.53) |
| Month 30 | 570 | 63.51 (61.89-65.13) | 143 | 59.54 (55.89-63.19) |
| Month 36 | 489 | 64.28 (62.56-66.00) | 117 | 60.26 (56.16-64.37) |
| Month 42 | 407 | 64.43 (62.50-66.35) | 101 | 59.55 (55.09-64.02) |
| Month 48 | 337 | 65.60 (63.48-67.72) | 81 | 59.84 (55.23-64.46) |
| Month 54 | 278 | 65.70 (63.39-68.00) | 60 | 60.35 (55.08-65.62) |
| Month 60 | 208 | 66.92 (64.25-69.59) | 42 | 60.01 (53.34-66.68) |

**Table 10**

| Baseline UA ≥ 8 | Average UA < 6 | | Average UA ≥ 6 | |
|---|---|---|---|---|
| | N | Mean (95% CI) | N | Mean (95% CI) |
| Baseline | 942 | 61.87 (60.73-63.01) | 429 | 63.17 (61.34-65.00) |
| Week 2 | 937 | 62.03 (60.84-63.22) | 422 | 63.18 (61.22-65.15) |
| Month 3 | 936 | 62.09 (60.91-63.28) | 425 | 63.02 (61.13-64.91) |
| Month 6 | 936 | 61.95 (60.74-63.16) | 426 | 62.56 (60.65-64.47) |
| Month 12 | 930 | 63.09 (61.87-64.32) | 421 | 62.75 (60.66-64.83) |
| Month 18 | 776 | 62.99 (61.62-64.36) | 343 | 61.52 (59.25-63.79) |
| Month 24 | 710 | 63.15 (61.69-64.61) | 284 | 62.08 (59.58-64.57) |
| Month 30 | 610 | 63.85 (62.28-65.43) | 237 | 60.68 (57.82-63.54) |
| Month 36 | 539 | 64.04 (62.38-65.70) | 191 | 61.32 (58.15-64.49) |
| Month 42 | 455 | 63.75 (61.92-65.59) | 166 | 60.90 (57.46-64.34) |
| Month 48 | 379 | 65.32 (63.28-67.37) | 130 | 61.54 (57.81-65.27) |
| Month 54 | 311 | 66.35 (64.11-68.58) | 99 | 62.30 (58.01-66.59) |
| Month 60 | 231 | 66.48 (63.94-69.02) | 69 | 61.06 (55.87-66.24) |

**[Table 11]**

| Gout ≥ 10 | Average UA < 6 | | Average UA ≥ 6 | |
|---|---|---|---|---|
| | N | Mean (95% CI) | N | Mean (95% CI) |
| Baseline | 686 | 65.79 (64.44-67.14) | 247 | 66.85 (64.41-69.28) |
| Week 2 | 678 | 65.90 (64.51-67.29) | 243 | 65.72 (63.06-68.37) |
| Month 3 | 682 | 65.15 (63.78-66.52) | 244 | 65.53 (63.05-68.01) |
| Month 6 | 680 | 65.11 (63.69-66.53) | 244 | 64.92 (62.53-67.31) |
| Month 12 | 677 | 66.55 (65.13-67.97) | 243 | 65.04 (62.45-67.64) |
| Month 18 | 562 | 66.31 (64.75-67.87) | 196 | 63.78 (60.85-66.70) |
| Month 24 | 500 | 66.05 (64.32-67.77) | 165 | 63.79 (60.66-66.92) |
| Month 30 | 435 | 66.94 (65.12-68.76) | 141 | 63.63 (60.01-67.26) |
| Month 36 | 386 | 66.58 (64.66-68.49) | 107 | 64.38 (60.10-68.65) |
| Month 42 | 323 | 67.99 (65.86-70.12) | 86 | 63.50 (58.94-68.06) |
| Month 48 | 268 | 69.86 (67.58-72.14) | 68 | 65.40 (60.50-70.31) |
| Month 54 | 213 | 70.12 (67.50-72.74) | 45 | 63.22 (56.77-69.67) |
| Month 60 | 162 | 71.57 (68.65-74.50) | 33 | 67.46 (59.11-75.80) |

Referring to Tables 7 to 11 and FIGS. 2A to 2E, if each patient group classified based on the five biomarkers maintained the serum uric acid concentration below 6, it was confirmed that the eGFR was maintained or rather increased. However, if the serum uric acid concentration was 6 or more, it was confirmed that the eGFR was decreased.

That is, it was confirmed that if febuxostat is administered to the patient group having the blood glucose level of 126 mg/dl or more, the diastolic blood pressure of 80 mmHg or lower, the systolic blood pressure of 130 mmHg or lower, the serum uric acid concentration or 8 mg/dl or more, or the gout history for 10 years or more to maintain the serum uric acid concentration below 6, the chronic kidney disease may be effectively prevented or treated. In Table 12, the risks of developing the kidney disease of the patients who had the serum uric acid concentration decreased to less than 6 and the patients who maintained the serum uric acid concentration at or above 6 were confirmed using the odds ratio, in both patient groups to which allopurinol or febuxostat was administered.

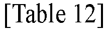

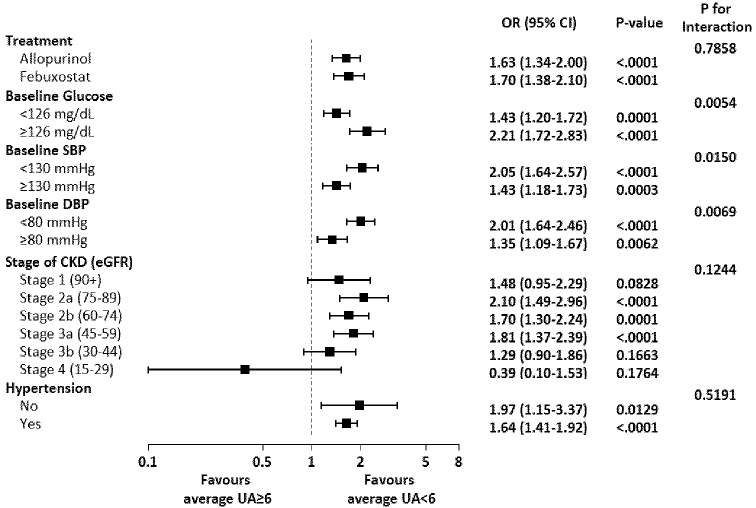

Referring to Table 12, it was confirmed that if the serum uric acid concentration is maintained below 6 in both the allopurinol administration group and the febuxostat administration group, the chronic kidney disease may be effectively prevented or treated.

In addition, it was confirmed that if allopurinol is administered to the patient group having the blood glucose level of 126 mg/dl or higher, the diastolic blood pressure of 80 mm/Hg or lower, or the systolic blood pressure of 130 mm/Hg or lower to maintain the serum uric acid concentration below 6, the chronic kidney disease may be effectively prevented or treated.

In addition, it was confirmed that the pharmaceutical composition according to one exemplary embodiment was more effective for the patients with the chronic kidney disease that is equal to or less severe than the moderate A chronic kidney disease (CKD 3A) with the eGFR of 45 ml/min/1.73 m² or more, and it was also more effective for the patients without hypertension.

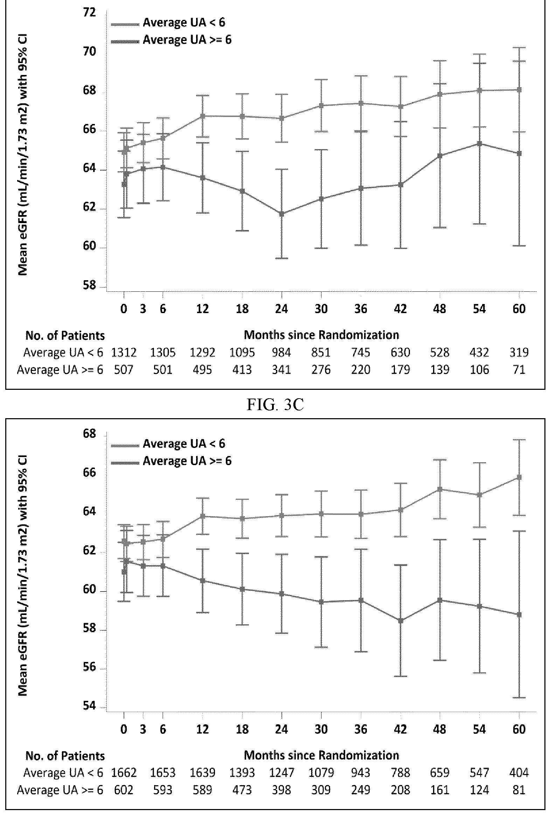

In Table 13, the change in eGFR over time for each patient group classified depending on the serum uric acid concentration in the allopurinol or febuxostat group having the blood glucose level of 126 mg/dl or higher is described, and is graphed in FIG. 3A. In Table 14, the change in eGFR over time for each patient group classified depending on the serum uric acid concentration in the allopurinol or febuxostat administration group having the systolic blood pressure of 130 mm/hg or lower is described, and is graphed in FIG. 3B. In Table 15, the change in eGFR over time for each patient group classified depending on the serum uric acid concentration in the allopurinol or febuxostat administration group having the diastolic blood pressure of 80 mm/hg or lower is described, and is graphed in FIG. 3C.

In Tables 13 to 15, all p-values were calculated by a linear mixed model using a randomized intercept model for repeated measurement analysis, and the p values for the interaction effect for the eGFR value and the average serum uric acid concentration for each visit were all 0.0001 or less.

**[Table 13]**

| Baseline blood glucose level ≥ 126 | Average UA < 6 | | Average UA ≥ 6 | |
|---|---|---|---|---|
| | N | Mean (95% CI) | N | Mean (95% CI) |
| Baseline | 903 | 64.00 (62.81-65.20) | 428 | 64.06 (62.12-66.01) |
| Week 2 | 896 | 63.76 (62.52-65.01) | 422 | 63.28 (61.28-65.28) |
| Month 3 | 895 | 63.73 (62.47-64.98) | 424 | 63.35 (61.32-65.39) |
| Month 6 | 898 | 63.55 (62.28-64.82) | 422 | 62.55 (60.56-64.54) |
| Month 12 | 889 | 64.33 (63.04-65.62) | 419 | 62.68 (60.59-64.76) |
| Month 18 | 735 | 63.94 (62.49-65.39) | 320 | 61.39 (58.99-63.79) |
| Month 24 | 636 | 64.54 (62.99-66.09) | 275 | 61.48 (58.88-64.08) |
| Month 30 | 547 | 63.48 (61.84-65.12) | 223 | 60.82 (57.98-63.65) |
| Month 36 | 482 | 63.95 (62.14-65.76) | 183 | 61.16 (57.82-64.49) |
| Month 42 | 398 | 63.08 (61.12-65.04) | 150 | 60.00 (56.31-63.69) |
| Month 48 | 318 | 64.90 (62.70-67.10) | 115 | 58.64 (54.77-62.51) |
| Month 54 | 259 | 65.53 (63.16-67.89) | 88 | 58.89 (54.50-63.27) |
| Month 60 | 188 | 66.26 (63.34-69.18) | 59 | 57.13 (51.76-62.49) |

**[Table 14]**

| SBP < 130 | Average UA < 6 | | Average UA ≥ 6 | |
|---|---|---|---|---|
| | N | Mean (95% CI) | N | Mean (95% CI) |
| Baseline | 1312 | 64.92 (63.91-65.93) | 507 | 63.26 (61.55-64.98) |
| Week 2 | 1309 | 65.14 (64.12-66.16) | 504 | 63.80 (62.04-65.56) |
| Month 3 | 1307 | 65.41 (64.38-66.45) | 502 | 64.07 (62.31-65.83) |
| Month 6 | 1305 | 65.63 (64.58-66.69) | 501 | 64.15 (62.43-65.87) |
| Month 12 | 1292 | 66.78 (65.71-67.85) | 495 | 63.60 (61.80-65.41) |
| Month 18 | 1095 | 66.77 (65.61-67.93) | 413 | 62.92 (60.89-64.96) |
| Month 24 | 984 | 66.67 (65.43-67.90) | 341 | 61.75 (59.46-64.03) |
| Month 30 | 851 | 67.33 (65.99-68.67) | 276 | 62.52 (59.99-65.06) |
| Month 36 | 745 | 67.44 (66.02-68.85) | 220 | 63.06 (60.15-65.98) |
| Month 42 | 630 | 67.27 (65.73-68.82) | 179 | 63.24 (59.98-66.50) |
| Month 48 | 528 | 67.90 (66.17-69.62) | 139 | 64.74 (61.05-68.44) |
| Month 54 | 432 | 68.10 (66.23-69.97) | 106 | 65.36 (61.24-69.49) |
| Month 60 | 319 | 68.14 (65.97-70.31) | 71 | 64.86 (60.10-69.61) |

**[Table 15]**

| DBP < 80 | Average UA < 6 | | Average UA ≥ 6 | |
|---|---|---|---|---|
| | N | Mean (95% CI) | N | Mean (95% CI) |
| Baseline | 1662 | 62.57 (61.70-63.43) | 602 | 61.00 (59.48-62.52) |
| Week 2 | 1652 | 62.45 (61.55-63.34) | 595 | 61.53 (59.94-63.12) |
| Month 3 | 1652 | 62.54 (61.64-63.43) | 595 | 61.32 (59.76-62.88) |
| Month 6 | 1653 | 62.67 (61.75-63.59) | 593 | 61.32 (59.74-62.90) |
| Month 12 | 1639 | 63.85 (62.93-64.78) | 589 | 60.55 (58.91-62.18) |
| Month 18 | 1393 | 63.73 (62.73-64.74) | 473 | 60.11 (58.27-61.96) |
| Month 24 | 1247 | 63.89 (62.81-64.96) | 398 | 59.87 (57.84-61.91) |
| Month 30 | 1079 | 63.97 (62.79-65.15) | 309 | 59.45 (57.12-61.78) |
| Month 36 | 943 | 63.96 (62.71-65.20) | 249 | 59.53 (56.89-62.18) |
| Month 42 | 788 | 64.18 (62.81-65.55) | 208 | 58.48 (55.62-61.35) |
| Month 48 | 659 | 65.25 (63.73-66.77) | 161 | 59.55 (56.44-62.66) |
| Month 54 | 547 | 64.96 (63.29-66.62) | 124 | 59.23 (55.80-62.66) |
| Month 60 | 404 | 65.86 (63.91-67.81) | 81 | 58.80 (54.51-63.09) |

Referring to Tables 13 to 15 and FIGS. 3A to 3C, if each patient group classified based on the three biomarkers maintained the serum uric acid concentration below 6, it was confirmed that the eGFR was maintained or rather increased. However, if the serum uric acid concentration was 6 or more, it was confirmed that the eGFR was decreased.

That is, it was confirmed that if allopurinol or febuxostat is administered to a patient group having the blood glucose level of 126 mg/dl or more, the diastolic blood pressure of 80 mmHg or lower, the systolic blood pressure of 130 mmHg or lower, or the serum uric acid concentration or 8 mg/dl or more to maintain the serum uric acid concentration below 6, the chronic kidney disease may be effectively prevented or treated.

It was confirmed from the above results that if the pharmaceutical composition according to one exemplary embodiment is administered to a subject having the blood glucose level of 126 mg/dl or more, the diastolic blood pressure of 80 mmHg or lower, the systolic blood pressure or 130 mmHg or lower, or the serum uric acid concentration of 6 mg/dl or more to maintain the serum uric acid concentration of subject below 6 mg/dl, a remarkably excellent effect may be shown in the prevention or treatment of the chronic kidney disease.

According to the present disclosure, a pharmaceutical composition is provided that may prevent or treat chronic kidney disease more effectively by selecting a subject group who may respond with an excellent effect when administered.

## Claims

1. A pharmaceutical composition for use in preventing or treating chronic kidney disease comprising: a therapeutically effective amount of allopurinol, febuxostat, or a pharmaceutically acceptable salt thereof as an active ingredient,
wherein the composition is administered to a subject having a serum uric acid concentration of 6 mg/dl or more to decrease the serum uric acid concentration of the subject to less than 6 mg/dl.

2. The pharmaceutical composition for use according to claim 1, wherein the subject has a blood glucose level of 126 mg/dl or more, a diastolic blood pressure of 80 mmHg or lower, or a systolic blood pressure of 130 mmHg or lower.

3. The pharmaceutical composition for use according to claim 2, wherein the subject has the blood glucose level of 126 mg/dl or more.

4. The pharmaceutical composition for use according to claim 2, wherein the subject has the diastolic blood pressure of 80 mmHg or lower and the systolic blood pressure of 130 mmHg or lower.

5. The pharmaceutical composition for use according to claim 1, wherein the active ingredient is the allopurinol or the pharmaceutically acceptable salt thereof, and the subject has no congestive cardiac failure.

6. The pharmaceutical composition for use according to claim 1, wherein the active ingredient is the febuxostat or the pharmaceutically acceptable salt thereof, and the subject has the serum uric acid concentration of 8 mg/dL or more.

7. The pharmaceutical composition for use according to claim 1, wherein the subject has the serum uric acid concentration of 7 mg/dl or more.

8. The pharmaceutical composition for use according to claim 1, wherein the subject has hyperuricacidemia or gout.

9. The pharmaceutical composition for use according to claim 1, wherein the subject has gout.

10. The pharmaceutical composition for use according to claim 9, wherein the active ingredient is the febuxostat or the pharmaceutically acceptable salt thereof, and wherein the gout has lasted 10 years or more.

11. The pharmaceutical composition for use according to claim 1, wherein the active ingredient is the allopurinol or the pharmaceutically acceptable salt thereof, and a dosage of the active ingredient is 100 mg to 600 mg per day based on an amount of the allopurinol.

12. The pharmaceutical composition for use according to claim 1, wherein the active ingredient is the febuxostat or the pharmaceutically acceptable salt thereof, and a dosage of the active ingredient is 20 mg to 120 mg per day based on an amount of the febuxostat.

13. The pharmaceutical composition for use according to claim 1, wherein the subject has an estimated glomerular filtration rate (eGFR) of 45 ml/min/1.73 m² or more.

14. The pharmaceutical composition for use according to claim 1,
wherein the active ingredient is the allopurinol or the pharmaceutically acceptable salt thereof,
wherein the active ingredient is administered to the subject having the eGFR of 60 ml/min/1.73 m² or more at a dose of 300 mg per day based on an amount of the allopurinol, and if the serum uric acid concentration of the subject is not decreased to less than 6 mg/dl at one month after the administration, the active ingredient is administered at a dose of 600 mg per day based on the amount of the allopurinol, and
wherein the active ingredient is administered to the subject having the eGFR of 30 ml/min/1.73 m² or more and less than 60 ml/min/1.73 m² at a dose of 200 mg per day based on the amount of the allopurinol, and if the serum uric acid concentration of the subject is not decreased to less than 6 mg/dl at one month after the administration, the active ingredient is administered at a dose which increased in increments of 100 mg per month up to a maximum of 400 mg per day based on the amount of the allopurinol.

15. The pharmaceutical composition for use according to claim 1,
wherein the active ingredient is the febuxostat or the pharmaceutically acceptable salt thereof, and
the active ingredient is administered at a dose of 40 mg per day based on an amount of the febuxostat, and if the serum uric acid concentration of the subject is not decreased to less than 6 mg/dl at 2 weeks after the administration, the active ingredient is administered at a dose of 80 mg per day based on the amount of the febuxostat.
